# EUROPEAN PATENT APPLICATION

(11) **EP 4 122 466 A1**
(43) Date of publication of application: **25.01.2023**
(21) Application number: 21382677.9
(22) Date of filing: 23.07.2021
(51) Int. Cl.: A61K 31/496, A61K 45/06, A61P 31/14

(54) **ENTRECTINIB AS ANTIVIRAL AGENT**

(71) Applicant: Fundació Institut Mar d'Investigacions Mèdiques (IMIM), 08003 Barcelona (ES); Fundació Hospital Universitari Vall d'Hebron - Institut de Recerca, 08035 Barcelona (ES)
(72) Inventor: SELENT, Jana, 08005 BARCELONA (ES); PERALTA GARCIA, Alejandro, 11300 LA LÍNEA DE LA CONCEPCIÓN (ES); BUZÓN GÓMEZ, María José, 08290 CERDANYOLA DEL VALLÉS (ES); GENESCÀ FERRER, Meritxell, 08012 BARCELONA (ES)
(74) Representative: ZBM Patents - Zea, Barlocci & Markvardsen

(57) **Abstract**

The present application refers to a compound of formula (I), also named entrectinib, or a pharmaceutically or veterinary acceptable salt thereof, for use in treating infections caused by enveloped viruses. Pharmaceutical or veterinary compositions of the compound of formula (I), as well as combinations thereof with other antiviral agents, for the same use are also contemplated.

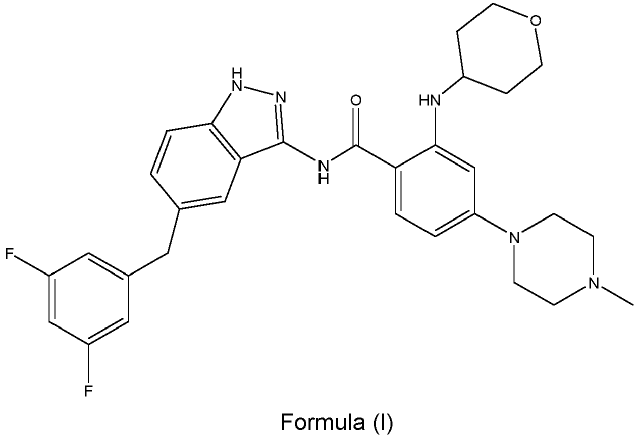

## Description

### Technical Field

The present invention relates to the use of entrectinib as an antiviral agent, in particular, for the treatment of COVID-19.

### Background Art

Severe acute respiratory syndrome coronavirus 2 (SARS-CoV-2) is the pathogen that causes the novel coronavirus disease-2019 (COVID-19). First detected in Wuhan, China, in December 2019 it quickly spread across the country and, by March 11 2020, the World Health Organization declared COVID-19 a global pandemic, present in almost every country across the globe. Since then, as of March 2021, 117 million people have been infected, counting 2.6 million deaths worldwide.

SARS-CoV-2 belongs to the broad family of viruses known as coronaviruses. Coronaviruses are a group of related RNA viruses that cause diseases in mammals and birds. They are enveloped viruses with a positive-sense single-stranded RNA genome and a nucleocapsid of helical symmetry. In humans and birds, they cause respiratory tract infections that can range from mild to lethal. Mild illnesses in humans include some cases of the common cold (which is also caused by other viruses, predominantly rhinoviruses), while more lethal varieties, like SARS-CoV-2, can be fatal.

Major pharmaceutical companies have focused on vaccine development as the primary response to COVID-19 outbreak. At the same time, several research groups and some pharmaceutical companies are looking for antiviral drugs, mainly targeting the spike protein, the RNA dependent RNA polymerase (RdRp or nsp12), and the main protease (Mpro or nsp5), to be used as non-immunological interventions. In the present pandemic situation, many researchers are exploring repositioning of approved drugs in order to find dug candidates at a lower cost and in a shorter time. However, most approaches do not go beyond computational screening and all of them rely on interactions between the drug candidate and the virus proteins. Only few of the numerous substances identified in said computational screenings have been shown to be effective in vitro, ex vivo or in vivo settings.

Among the candidates for treating SARS-CoV-2, some tyrosine kinase inhibitors have been suggested for theoretically blocking the fusion between viral envelope and endosomal membrane. Very recently, Cagno et al (Basic Clin. Pharmacol. Toxicol. 128, 621-624 (2021)) reported the in vitro activity against SARS-CoV-2 of three of said tyrosine kinase inhibitors, namely, nilotinib, imatinib and dasatinib. These authors reported that imatinib and dasatinib do not show antiviral efficacy, while nilotinib did show in vitro antiviral effect with Vero-E6 cells and Calu-3 cells.

Moreover, the approaches mentioned above (vaccines and antivirals targeting virus proteins) are at permanent risk of becoming ineffective with the evolution of viral genomes. Unfortunately, such an evolution is essentially a given. Viruses evolve very quickly, more so when having such a high expansion rate as is currently the case for SARS-CoV-2. Many of SARS-CoV-2 variants already exist as a result of mutations in the virus proteins. Although most mutations in SARS-CoV-2 genome are expected to be either deleterious and swiftly purged or relatively neutral, a small proportion will affect functional properties and may alter infectivity, disease severity or interactions with host immunity. Indeed, several emerging mutations have been described that show substantially increased infectivity. The World Health Organization has currently declared four variants of concern, which are as follows:
Alpha: Lineage B.1.1.7 emerged in the United Kingdom in September 2020, with evidence of increased transmissibility and virulence. Notable mutations include N501Y and P681H. An E484K mutation in some lineage B.1.1.7 virions has been noted and is also tracked by various public health agencies.

Beta: Lineage B.1.351 emerged in South Africa in May 2020, with evidence of increased transmissibility and changes to antigenicity, with some public health officials raising alarms about its impact on the efficacy of some vaccines. Notable mutations include K417N, E484K and N501Y.

Gamma: Lineage P.1 emerged in Brazil in November 2020, also with evidence of increased transmissibility and virulence, alongside changes to antigenicity. Similar concerns about vaccine efficacy have been raised. Notable mutations also include K417N, E484K and N501Y.

Delta: Lineage B.1.617.2 emerged in India in October 2020. There is also evidence of increased transmissibility and changes to antigenicity.

The emergence of mutated SARS-CoV-2 variants with increased transmission rates, severe disease progressions, and/or resistance to vaccinal campaigns and drugs poses a serious threat to global health. In this respect, there is an urgent need to provide effective antiviral drugs with increased resistance to SARS-CoV-2 evolution.

### Summary of Invention

The inventors have found that entrectinib, a tyrosine kinase inhibitor that has been recently approved for the treatment of cancer, shows surprisingly good antiviral properties against SARS-CoV-2 in in vitro experiments. Moreover, as shown in the examples below, the antiviral effect translates to an ex vivo setting, wherein entrectinib showed a potent decrease of cell infection without inducing significant cell death, i.e. no toxicity (EC50 < 1 µM). In contrast, positive control apilimod produced a lower decrease in infection rate. Surprisingly, the present inventors found that nilotinib showed a significantly lower activity in the same ex vivo setting (EC50 > 15 µM) when compared to entrectinib.

Interestingly, the inventors also found that entrectinib, in addition to blocking the interface between the receptor binding domain of SARS-CoV-2 and the ACE2 host cell receptor (specific antiviral effect), interferes with the viral membrane (un-specific antiviral effect). Without being bound by theory, it is thought that the combined specific and unspecific antiviral effects afforded by entrectinib result in a superior antiviral activity and, perhaps even more importantly, an advantage in facing virus evolution and the emergence of new variants. This is because the viral membrane is not prone to mutations. Entrectinib is therefore less susceptible to loss of effectiveness against new variants and could be used for treating patients that are refractive to first line COVID-19 treatment.

Moreover, given this additional mechanism, entrectinib also shows antiviral activity against other enveloped viruses. This unspecific antiviral effect has been evidenced with VSV.G virus - an envelope virus lacking the SARS-CoV spike protein- for which entrectinib also demonstrated an antiviral effect (Figure 7).

Thus, a first aspect of the invention refers to a compound of formula (I), or a pharmaceutically or veterinary acceptable salt thereof, for use in treating infections caused by enveloped viruses. This aspect could also be envisioned as a compound of formula (I) or pharmaceutically or veterinary acceptable salt thereof, for use as antiviral.

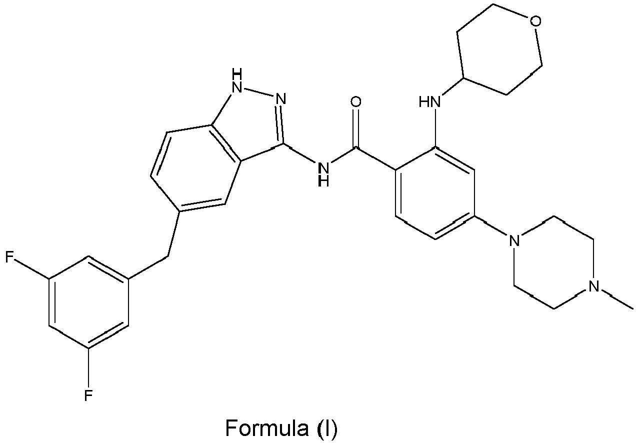

This first aspect may also be formulated as use of a compound of formula (I) or pharmaceutically or veterinary acceptable salt thereof, for the preparation of a medicament for treating infections caused by enveloped viruses. The application also refers to a method treating infections caused by enveloped viruses, which comprises administering to a subject in need thereof, including a human, a therapeutically effective amount of a compound of formula (I) or a pharmaceutically or veterinary acceptable salt thereof, together with one or more pharmaceutically acceptable excipients or carriers.

In a second aspect, the invention provides a pharmaceutical or veterinary composition comprising a therapeutically effective amount of a compound of formula (I), or a pharmaceutically or veterinary acceptable salt thereof, together with one or more pharmaceutically or veterinary acceptable excipients or carriers, for use in treating infections caused by enveloped viruses.

The compound of formula(I) or compositions thereof for use as defined in the first and second aspects may be used in combination with other antivirals. Combinatorial antiviral therapy has been shown to be effective for treating several viruses. Complementary or synergic effects may be conveniently achieved by combining two or more antiviral agents, for example, by combining two antiviral agents having different mechanism of action.

Thus, a third aspect refers to a combination comprising: a) compound of formula (I) or pharmaceutically or veterinary acceptable salt thereof, and b) a second antiviral pharmaceutical or veterinary active ingredient that is different from the compound of formula (I), or pharmaceutically or veterinary acceptable salt thereof.

The drug combinations of the invention may be formulated in different types of compositions/kits of parts. Thus, a fourth aspect of the invention relates to a single pharmaceutical or veterinary composition which comprises a therapeutically effective amount of: a) a compound of formula (I), or a pharmaceutically or veterinary acceptable salt thereof; and b) a second antiviral pharmaceutical or veterinary active ingredient that is different from the compound of formula (I) or pharmaceutically or veterinary acceptable salt thereof, together with one or more pharmaceutically or veterinary acceptable excipients or carriers.

A fifth aspect refers to a package or kit of parts comprising: i) a first pharmaceutical composition or veterinary which comprises a therapeutically effective amount of a compound of formula (I), or a pharmaceutically or veterinary acceptable salt thereof, together with one or more pharmaceutically or veterinary acceptable excipients or carriers; and ii) a second pharmaceutical or veterinary composition which comprises a therapeutically effective amount of a second antiviral pharmaceutical or veterinary active ingredient or a pharmaceutically or veterinary acceptable salt thereof, together with one or more pharmaceutically or veterinary acceptable excipients or carriers; wherein compositions i) and ii) are separate compositions.

Further, the combination of the invention may be used in treating infections caused by enveloped viruses. Thus, a sixth aspect of the invention relates to the combination, the single pharmaceutical or veterinary composition, or the package or kit of parts as previously defined, for use in treating an infection caused by an enveloped virus.

A seventh aspect of the invention relates to a compound of formula (I), or a pharmaceutically or veterinary acceptable salt thereof, for use in combination therapy in the treatment of infections caused by an enveloped virus SARS-CoV-2, wherein the compound of formula (I), or a pharmaceutically or veterinary acceptable salt thereof, is to be administered simultaneously, concurrently, separately or sequentially within a therapeutic interval with a second antiviral pharmaceutical or veterinary active ingredient or a pharmaceutically or veterinary acceptable salt thereof that is different from the compound of formula (I).

### Brief Description of Drawings

Fig. 1: Database creation process.
Fig. 2: Most relevant contacts in the RBD-ACE2 interface. (A) Contact heatmap: residues with at least 50% of contacts during the molecular dynamics simulation are plotted. Most relevant contacts with ≥ 90% of contacts are marked with asterisk. (B) Structural representation of the RBD and ACE2 depicting most relevant contacts (> 90%) in licorice.
Fig 3: Membrane intercalation. (A) Octanol/water partition coefficient (log P). The LogP has been computed as h_logP, a molecular descriptor implemented in the MOE software. (B) Molecular dynamics simulations reveal membrane intercalation of drugs with high LogP computed as number of ligand atoms which are in a distance < 3 Å from the lipid tails/nonpolar cholesterol, (C) Atomistic snapshots of drug intercalation into the viral membrane.
Fig 4: SARS-CoV-2 pseudovirus entry assay.
Fig 5: Antiviral assay in Human Lung Tissue (HTL) cells. The area with diagonal lines reflects the cell infection [%] with increasing concentration whereas the area with horizontal lines monitors cell deaths [%].
Fig 6: Cell entry assay for the VSV.G virus.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The first aspect of the invention refers to entrectinib for use as antiviral agent in the treatment of infections caused by enveloped viruses.

For the purposes of the invention, the term "antiviral" or "antiviral agent" as used herein refers to a compound or drug that is used to treat viral infections. Antivirals have different mechanisms of actions and many target stages in the viral life cycle. Non-limiting target stages in the viral life cycle are: viral attachment to host cell, uncoating, synthesis of viral mRNA, translation of mRNA, replication of viral RNA and DNA, maturation of new viral proteins, budding, release of newly synthesized virus, and free virus in body fluids.

### The compound of formula (I)

The compound of formula (I) is also known as entrectinib (CAS Number 1108743-60-7).

This pharmaceutical active ingredient has been recently approved in several countries for treating cancer, in particular, ROS1-positive non-small cell lung cancer and NTRK fusion-positive solid tumors. It is a selective tyrosine kinase inhibitor, of the tropomyosin receptor kinases A, B and C, C-ros oncogene 1 and anaplastic lymphoma kinase. This drug is currently sold under the brand name Rozlytrek.

There is no limitation on the type of salt of the compound of formula (I) that can be used, provided that these are pharmaceutically acceptable when they are used for therapeutic purposes. The term "pharmaceutically acceptable salts", embraces salts commonly used to form alkali metal salts and to form addition salts of free acids or free bases.

The preparation of pharmaceutically acceptable salts of the compound of formula (I) can be carried out by methods known in the art. Non-limiting examples of pharmaceutically acceptable salts of entrectinib which can be used for the purposes of the present invention include benzoate, acetate, propionate, butyrate, pentanoate, hexanoate, heptanoate, octanoate, lactate, maleate, oxalate, fumarate. tartrate, malate, citrate, nicotinate, salicylate, pamoate, hemipamoatye, ascorbate, methanesulfonate, ethanesulfonate, benzenesulfonate, p-toluenesulfonate, camphorsulfonate, hydrochloride, hydrobromide, hydroiodide, sulfate, nitrate, phosphate, hydrogenphosphate, dihydrogenphosphate, carbonate, hydrogencarbonate, perchlorate, aspartate or glutamate.

The compound of formula (I) may be in crystalline form either as free solvation compounds or as solvates (e.g. hydrates). All these forms are within the scope of the present invention. Methods of solvation are generally known within the art. In general, the solvated forms with pharmaceutically acceptable solvents such as water, ethanol and the like are equivalent to the unsolvated form for the purposes of the invention.

In all embodiments of the invention referring to the compound of formula (I), its pharmaceutically or veterinary acceptable salts are always contemplated even if not specifically mentioned.

### Compositions

A second aspect of the invention refers to a pharmaceutical or veterinary composition comprising a therapeutically effective amount of a compound of formula (I), or salts thereof, together with excipients or carriers for use in treating infections caused by enveloped viruses.

The expression "therapeutically effective amount" as used herein, refers to the amount of a compound that, when administered, is sufficient to prevent development of, or alleviate to some extent, one or more of the symptoms of the disease which is addressed. The specific dose of the compound of the invention to obtain a therapeutic benefit may vary depending on the particular circumstances of the individual patient including, among others, the size, weight, age and sex of the patient, the nature and stage of the disease, the aggressiveness of the disease, and the route of administration.

In a particular embodiment, optionally in combination with one or more features of the embodiments described above or below throughout all the description, the daily dose of the compound of formula (I) ranges from 50 to 4000, for example, from 100 to 2000 mg, for example, from 100 to 1000, or from 200 to 1000 mg, or from 300 to 900 mg, or from 300 to 800 mg, or from 400 to 800 mg.

The expression "pharmaceutically or veterinary acceptable excipients or carriers" refers to pharmaceutically or veterinary acceptable materials, compositions or vehicles. Each component must be pharmaceutically or veterinary acceptable in the sense of being compatible with the other ingredients of the pharmaceutical or veterinary composition. It must also be suitable for use in contact with the tissue or organ of humans and animals without excessive toxicity, irritation, allergic response, immunogenicity or other problems or complications commensurate with a reasonable benefit/risk ratio.

The election of the pharmaceutical or veterinary formulation will depend upon the nature of the active compound and its route of administration. Any route of administration may be used, for example oral, parenteral and topical administration.

For example, the pharmaceutical or veterinary composition may be formulated for oral administration and may contain one or more physiologically compatible carriers or excipients, in solid or liquid form. These preparations may contain conventional ingredients such as binding agents, fillers, lubricants, and acceptable wetting agents.

In another example the pharmaceutical or veterinary composition may be formulated for administration by inhalation and may contain one or more appropriate carriers or excipients, such as sugars, lipids, amino acids, surfactants, polymers and absorption enhancers. Non-limitative inhalation systems are nebulizers, pressurized metered-dose inhalers and dry powder inhalers. Typically, the active pharmaceutical ingredient in said formulations is in aerosol form.

The pharmaceutical or veterinary composition may be formulated for parenteral administration in combination with conventional injectable liquid carriers, such as water or suitable alcohols. Conventional pharmaceutical or veterinary excipients for injection, such as stabilizing agents, solubilizing agents, and buffers, may be included in such compositions. These pharmaceutical or veterinary compositions may be injected intramuscularly, intraperitoneally, or intravenously.

The pharmaceutical or veterinary compositions may be in any form, including, among others, tablets, pellets, capsules, aqueous or oily solutions, suspensions, emulsions, aerosols, or dry powdered forms suitable for reconstitution with water or other suitable liquid medium before use, for immediate or retarded release.

The appropriate excipients and/or carriers, and their amounts, can readily be determined by those skilled in the art according to the type of formulation being prepared.

### Indication

The compound or compositions as described above are for use in treating viral infections caused by enveloped viruses. The term "treating" does not exclude prophylactic treatment. In most embodiments, however, entrectinib is for use in treating or controlling an on-going viral infection.

Enveloped viruses are viruses that have an outer wrapping or envelope. This envelope comes from the infected cell, or host, in a process called "budding off." During the budding process, newly formed virus particles become "enveloped" or wrapped in an outer coat that is made from a small piece of the cell's plasma membrane. Non-limiting examples of enveloped viruses within the scope of the invention are coronaviruses, herpesviruses, poxviruses, hepadnaviruses, asfarviridae, flaviviruses, alphaviruses, togaviruses, hepatitis D virus, orthomyxoviruses, paramyxoviruses, rhabdovirus, bunyaviruses, filoviruses, and retroviruses. In a particular embodiment of the first and second aspects of the invention, optionally in combination with one or more features of the embodiments described above or below throughout all the description, the enveloped virus is a coronavirus, for example selected from the group consisting of SARS-CoV-2, SARS-CoV-1, MERS-CoV, HCoV-OC43, HCoV-HKU1, HCoV-229E, HCoV-NL63, and Indiana vesiculovirus, formerly Vesicular stomatitis Indiana virus. Accordingly, in particular embodiments, optionally in combination with one or more features of the embodiments described above or below throughout all the description, the present invention encompasses treating a disease selected from the group consisting of a common cold, severe acute respiratory syndrome (SARS), middle East respiratory syndrome (MERS), and coronavirus disease 2019 (COVID-19). In a more particular embodiment, optionally in combination with one or more features of the embodiments described above or below throughout all the description, the enveloped virus is SARS-CoV-2 and the disease is COVID-19.

The present invention contemplates treating humans as well as other animals, preferably, mammals, in particular, livestock and/or pets, such as cattle, sheep, pigs, goats, horses, donkeys, mules, oxen, llamas, camels, cats, or dogs. In particular embodiments, optionally in combination with one or more features of the embodiments described above or below throughout all the description, the subject to be treated in a human, for example for treating COVID-19 in humans.

As explained above, the compound of formula (I) acts by a dual mechanism. On the one hand the compound has a specific antiviral effect by blocking the interface between the receptor binding domain of SARS-CoV-2 and the ACE2 host cell receptor. On the other hand, the compound has an un-specific antiviral effect which comprises interfering with the viral membrane. Without wishing to be bound by theory, it is hypothesized that entrectinib may intercalate into the viral membrane inhibiting viral entry possibly as a result of hindering virus-cell fusion. This is thought to happen due to the hydrophobicity of the compound, which can be approximated using the octanol/water partition coefficient (logP). Therefore, in one embodiment, optionally in combination with one or more features of the various embodiments described above or below throughout all the description, the treatment of the infections is, at least in part, mediated by disrupting the viral membrane, in particular, by disrupting viral membrane dynamics that are required for virus-cell fusion and subsequent cell entry.

This un-specific mode of action is, moreover, not affected by virus evolution, and, thus, provides the advantage that the present therapy is not as susceptible to virus mutations as other conventional antiviral agents, in particular those targeting relevant virus proteins. As a result, patients that are refractive to other antiviral therapies, in particular those that are susceptible of losing effectiveness as a result of virus evolution, may be successfully treated with the present therapy. In one embodiment, optionally in combination with one or more features of the various embodiments described above or below throughout all the description, the treatment comprises administering the compound to a patient that is refractory to treatment with a previous antiviral drug. The previous antiviral drug could be any drug prescribed for treating the viral infection, in particular those that act through a virus-specific mechanisms. For example, in the case of COVID-19 infection, several antiviral drugs are being used or are under investigation that target SARS-CoV-2 proteins, including viral RNA polymerase inhibitors (e.g. remdesivir), viral protein synthesis inhibitors (e.g. ritonavir, lopinavir), inhibitors of viral entry (e.g. hydroxychloroquine, chloroquine), immunomodulators (e.g. nitazoxanide, ivermectin), Inhibitors of the cytoplasmic replication (e.g. plitidepsin), and PF-07321332. In particular embodiments, the treatment comprises administering the compound of formula I to a patient that is refractory to a previous treatment with an antiviral selected from the group consisting of a viral RNA polymerase inhibitor, a viral protein synthesis inhibitor, an inhibitor of viral entry, an immunomodulator, an inhibitors of the cytoplasmic replication, and PF-07321332. In particular, the antiviral is selected from the group consisting of remdesivir, ivermectin, plitidepsin, lopinavir, ritonavir, chloroquine, hydroxychloroquine, nitazoxanide, and PF-07321332.

In one embodiment, optionally in combination with one or more features of the various embodiments described above or below throughout all the description, the compound of formula (I) is for treating infections caused by new variants of SARS-CoV-2.

In one embodiment, optionally in combination with one or more features of the various embodiments described above or below throughout all the description, the compound of formula (I) is in an amount sufficient to cause disruption of the virus membrane. For example, a sufficient amount may be a daily dose ranging 50 to 4000, for example, from 100 to 2000 mg, for example, from 100 to 1000, or from 200 to 1000 mg, or from 300 to 900 mg, or from 300 to 800 mg, or from 400 to 800 mg.

### Combinations with other antivirals

The third aspect of the invention refers to combinations of compound of formula (I) with another pharmaceutical or veterinary active ingredients. Combination of the compound of formula (I) with more than one pharmaceutical or veterinary active ingredient is also contemplated. In some embodiments, optionally in combination with one or more features of the various embodiments described above or below throughout all the description, the other pharmaceutical or veterinary active ingredient is another antiviral agent.

The active agents can be formulated in a single pharmaceutical or veterinary composition together with excipients and carrier, or may be formulated in separate compositions, each containing excipients and carriers. When formulated in separate compositions, they can be included in a kit of parts as defined in the fourth aspect of the invention.

All embodiments referring to the compound of formula (I) and compositions thereof disclosed above also apply to the combinations or kits of parts of the third and fourth aspects of the invention.

Moreover, the combination and kit of parts of the third and fourth aspects of the invention may be for use in treating infections caused by enveloped viruses. Thus, in one embodiment, optionally in combination with one or more features of the various embodiments described above or below throughout all the description, the combinations or kits of parts of the third and fourth aspects of the invention are for use in treating infections caused by enveloped viruses. All embodiments referring to the indication as described above also apply to the combinations or kits of parts. In one particular embodiment, the combination and kit of parts of the third and fourth aspect of the invention are for use in treating COVID-19.

In some particular embodiments, optionally in combination with one or more features of the various embodiments described above or below throughout all the description, the other pharmaceutical or veterinary active ingredient is an antiviral agent selected from the group consisting of a viral RNA polymerase inhibitor, a viral protein synthesis inhibitor, an inhibitor of viral entry, an immunomodulator, an inhibitors of the cytoplasmic replication, and PF-07321332. In particular, the antiviral is selected from the group consisting of remdesivir, ivermectin, plitidepsin, lopinavir, ritonavir, chloroquine, hydroxychloroquine, nitazoxanide, and PF-07321332.

The combination therapy of the present invention may include sequential, simultaneous, separate, or co-administration of the above compounds of the invention, wherein the therapeutic effects of the first administered has not entirely disappeared when the subsequent compounds are administered.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

### Materials and methods

### RBD-ACE2 interface examination

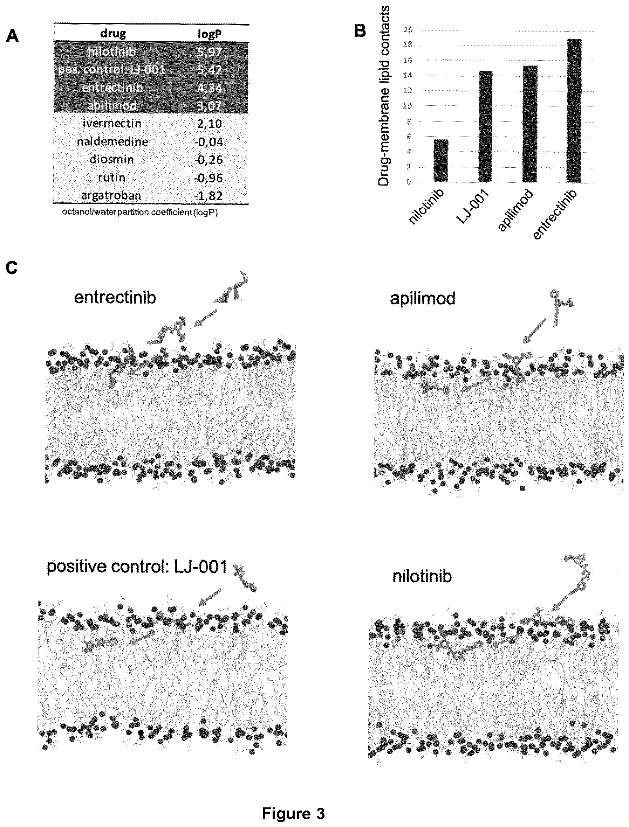

To find relevant regions that mediate contacts in the interface between the RBD and the ACE2, a 10 µs simulations of the RBD-ACE2 complex (PDB 6M17) from D.E. Shaw laboratory was analysed (Simulation ID: DESRES-ANTON-10905033, http://www.deshawresearch.com/resources sarscov2.html) using the GetContacts 2.0 software (https://getcontacts.github.io/). Obtained contact frequencies are plotted in Figure 3A.

### Database creation

A database of every FDA and EMA approved drug was created for the subsequent analysis. For this purpose, DrugBank and PubChem databases were used, merging both of them in a single database in SDF format. Redundancies in both databases were avoided by identifying common IDs in the merging step. Intermediate drug metabolites were also included in the merged database with the use of ZINC15 HMDB Drug Metabolites database (J. Chem. Inf. Model. 55, 2324-2337 (2015)). A total of 5849 compounds formed the database after redundancies were deleted.

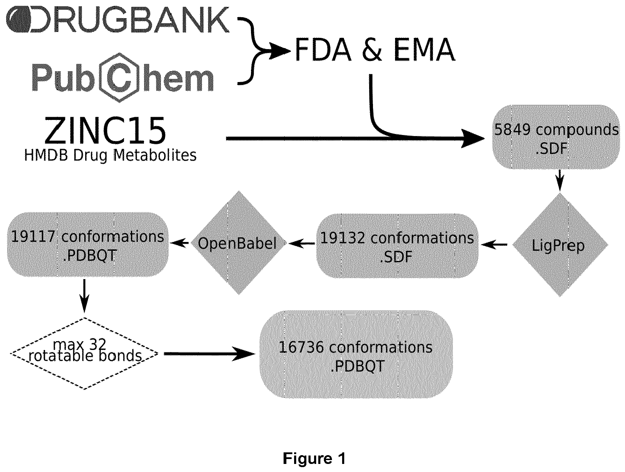

Different conformations for each compound were found using LigPrep software (Schrödinger Release 2021-1: LigPrep, Schrödinger, LLC, New York, NY, 2021). This resulted in a total of 19132 conformations, with an average of 3.27 conformations per compound. Next, every conformation was converted to PDBQT format using OpenBabel 2.4.0 software (J. Cheminformatics 3, 33 (2011)). Some compounds could not be converted into this format, losing 15 conformations for a total 19117. In the last step, we removed compounds that cannot not be properly docked by the molecular docking software, i.e. > 32 rotatable bonds as recommended by the software authors, yielding a total of 16736 files (Figure 1).

### Molecular Docking

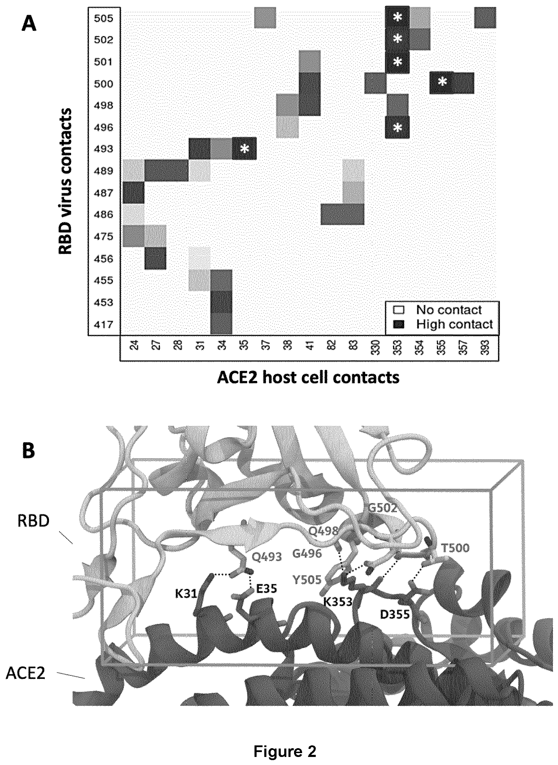

For the molecular docking, AutoDock Vina 1.1.2 software (J. Comput. Chem. 31, 455-461 (2010)) was utilized. First, RBD and ACE2 proteins were prepared and converted into PDBQT format using AutoDock Tools 1.5.6 software (Nat. Protoc. 11, 905-919 (2016)). Then, the docking was performed against the RBD region and the ACE2 protein, both in an unbound state from a simulation from D.E. Shaw laboratory (DESRES-ANTON-10895671 based on PDB 6VW1, replicate 000151, first frame) using the 19117 conformations database. The docking region was set to cover the most important residues in the interface found in the previous analysis step, with its dimensions being 18Å x 30Å x 40Å (see docking box in Figure 2B). This process was made using an exhaustiveness parameter of 10 and finding the best 5 poses. Top hits were analyzed using VMD 1.9.3 software (J. Mol. Graph. 14, 33-38 (1996)).

### Molecular Dynamics simulations

*System building, parametrization and simulation.* All systems were built using the HTMD platform (Doerr S, Harvey MJ, Noé F, De Fabritiis G. HTMD: High-Throughput Molecular Dynamics for Molecular Discovery. J Chem Theory Comput. 2016;12(4):1845-1852). Protein parameters were obtained from CHARMM36 (Klauda JB, Venable RM, Freites JA, et al. Update of the CHARMM all-atom additive force field for lipids: validation on six lipid types. J Phys Chem B. 2010;114(23):7830-7843) and CHARMM36m (Huang J, Rauscher S, Nawrocki G, et al. CHARMM36m: an improved force field for folded and intrinsically disordered proteins. Nat Methods. 2017;14(1):71-73) force fields. Ligand parameters were obtained from the CgenFF forcefield using the paramchem service. For NPT (constant pressure, 1.01325 bar) simulations we have used a timestep of 2fs, pressure was maintained using the Berendsen barostat. For NVT (constant volume) simulations we have used a timestep of 4fs. Simulations were carried out at a temperature of 300 K (maintained using the Langevin thermostat) in periodic boundary conditions. The charge of the systems was kept at 0, using a 0.15 concentration of NaCl ions. The non-bonded interaction cutoff was set at 9 Å. A smooth switching function for the cut-off was applied, starting at 7.5 Å. Long-distance electrostatic forces were calculated using the Particle Mesh Ewald algorithm, with A grid spacing of 1 Å. The bond lengths of hydrogen atoms were kept constrained using the RATTLE algorithm.

For simulations we have used the ACEMD package (Harvey MJ, Giupponi G, Fabritiis GD. ACEMD: Accelerating Biomolecular Dynamics in the Microsecond Time Scale. J Chem Theory Comput. 2009;5(6):1632-1639). Resulting simulations were analyzed using the VMD 1.9.3 software (Humphrey, W., Dalke, A. & Schulten, K. VMD: Visual molecular dynamics. J. Mol. Graph. 14, 33-38 (1996)).

*Spontaneous association of ligands.* To assess preferred ligand recognition points to the RBD of the spike protein, we have used the structure of the RBD in complex with the human ACE2 receptor (PDB code: 6VW1). The proteins underwent a curation process which included modelling of missing atoms and side-chains, removal of glycosylation and the placement of a zinc ion in the established binding site. Such a system was then protonated using propka at a pH of 7.4, and then solvated with TIP3P waters. The systems were first equilibrated in NPT conditions for 20 ns with restraints applied to ligand heavy atoms and the protein backbone. After this step each system was simulated for 500 ns in NVT conditions. The preferred ligand binding spots were assessed by plotting a volumetric occupancy map using VMD 1.9.3. software.

*Ligand intercalation into membrane.* To asses the the capability of ligands to intercalate with the lipid bilayer, we have generated a human-like membrane, using previously established protocols (Casalino L, Gaieb Z, Goldsmith JA, et al. Beyond Shielding: The Roles of Glycans in the SARS-CoV-2 Spike Protein. ACS Cent Sci. 2020;6(10):1722-1734). For each system five ligands were placed in the vicinity of the membrane, however in a distance preventing formation of direct contacts. The systems were equilibrated for 20ns, with restraints applied to ligand heavy atoms. Such an equilibrated system was then simulated in 3 independent replicates for 1us. Within the resulting frames we have quantified in each frame the number of ligand atoms which are in a distance smaller than 3A from the lipid tails/non-polar cholesterol atoms forming the membrane.

### Plasmids & Cell lines

Lenti X 293T cell line (632180) was purchased from Takara Bio. Third generation lentivirus packaging plasmids - pLenti CMV Puro Luc (17477), pMDLg.pRRE (12251), pRSV.REV (12253), pMD2.G (12259) were purchased from Addgene. Full length SARS-CoV-2 Spike plasmid (VG40589-UT) was purchased from Sino Biological. The Spike ORF was subcloned into pcDNA3.1(+) vector via Gibson Assembly (NEB). Subsequently, a truncated version without the C terminal 21 amino acids, Spike_CTR plasmid was generated via Q5 Site Directed Mutagenesis (NEB).

### SARS-CoV-2 pseudovirus production

Lenti X 293T were seeded in a T175 flask and topped up with media (DMEM + 10% FBS + 1mM Sodium Pyruvate) for a cell density of 6x10⁴ per cm². Next day, three types of pseudoviruses were made: spike (plasmids pLenti Luc 20µg, pMDLg.pRRE 20µg, pRSV.REV 9.5µg, spike_CTR 10.5µg. Molar ratio of 1 for each plasmid except for spike_CTR, molar ratio: 0.5), VSV.G (pLenti Luc 20µg, pMDLg.pRRE 20µg, pRSV.REV 9.5µg, pMD2.G 6.5µg. Molar ratio of 1 for each plasmid except for pMD2.G, molar ratio: 0.5) and Δspike (p Lenti Luc 20µg, pMDLg.pRRE 20µg, pRSV.REV 9.5µg. Molar ratio of 1 for each plasmid). For the creation of each type of pseudovirus the plasmid DNA was added to a total amount of 1 ml sterile OptiMEM. Then PEI was added at a 1:3 mass ratio to a total amount of 1 ml sterile OptiMEM. PEI solution was then mixed with plasmid solution for a total of 2ml and left for 20min at room temperature. The mixed solution was then transferred to the T175 flasks and incubated in a BSL2 incubator at 37°C and 5% CO₂ for 72 hours. After incubation, one sterile 50ml tube per T175 flask was labeled and dispensed 5ml of sterile 3M NaCl for a final 0.3M concentration. Media supernatant from each flask was collected and filtered via 0.45µm sterile syringe filter in the corresponding 50ml tube. PEG8K was added per tube for a final 10% concentration and a total volume around 50ml. The solution was mixed and incubated overnight at 4°C. Next day, tubes were centrifuged at 4°C for 45min at 1500g. The supernatant was decanted into a beaker with Incidin Plus, leaving a pellet at the bottom of the tube. 3.5ml of complete media was added to teach tube to resuspend the pellet. Finally, a total amount of around 3.75ml per 50ml tube will be distributed in 2ml storage tubes not exceeding 1.8ml per tube. These tubes were stored at -80°C overnight and then transferred to a storage box.

### SARS-CoV-2 pseudovirus assay

A total of 3x10⁴ Lenti X 293T cells were seeded per well in white solid bottom 96 well plates that were either Poly L Lysine or Collagen coated with fresh media (DMEM + 10% FBS + 1mM Sodium Pyruvate). Plates are transferred to an incubator at 37°C, 5% CO₂ for 18-22 hours. For the transfection of hACE2 and hTMPRSS2a, the DNA was diluted in OptiMEM along with Lipofectamine 2000 in the following concentrations per well: hACE2 150ng, hTMPRSS2 15ng, Lipofectamine 2000 50µl, OptiMEM 2335µl. The mix was incubated at room temperature for 20min. Then, 50µl of the solution was added to each well. The plates were placed in a TC incubator for 6-8 hours. After that, media in all wells was replaced with 100µl completed media and the plates returned to the TC incubator overnight. Next day, 3 hours before transduction, cells were treated with 20µl of the compounds in study at different concentrations: 10⁻⁵M, 10⁻⁶M, 10⁻⁷M, 10⁻⁸M, 10⁻⁹M and 10⁻¹⁰M. They were also treated with anti ACE2 antibody as a positive control. Viruses were thawed 1.5 hours before transduction. Then, 100µl of the respective virus was dispensed to the wells and, last, 20 µl of the respective compound was added again to compensate for the dilution by viral solution addition. 48 hours after the transduction the plates were transferred to room temperature for 10min. The media was aspirated and washed with 200µl sterile PBS. Next, 50 µl of PBS and 50µl of One Glo Ex substrate were added per well and the plates were sealed and incubated in the dark for 10 min. Finally, plates were analyzed on the Pherastar 80 using the LUM Plus program with a focus adjustment of 10mm, top mode selected and 1s of integration time.

### Antiviral assays in Human Lung Tissue (HTL) cells

The antiviral assays were performed as recently described (Grau-Expósito, J. et al. Novel Human Lung Tissue Model for the Study of SARS-CoV-2 Entry, Inflammation and New Therapeutics. bioRxiv 2021.04.21.440731 (2021)). Briefly, non-neoplastic areas of lung tissues were obtained from patients undergoing thoracic surgical resection at the Thoracic Surgery Service of the Vall d'Hebron University Hospital. Study protocol was approved by the Ethical Committee (Institutional Review Board number PR(AG)212/2020). Tissue was enzymatically digested with 5 mg/ml collagenase IV (Gibco) and 100 µg/ml of DNase I (Roche) for 30 min at 37°C and 400 rpm and, then, mechanically digested with a pestle. The resulting cellular suspension was washed twice with PBS and resuspended in fresh medium (RPMI 1640 supplemented with 5% FBS, 100 U/ml penicillin, and 100 µg/ml streptomycin) and DNase I to dissolve cell aggregates. Cell number and viability were assessed with LUNA^{™} Automated Cell Counter (Logos Biosystems). Triplicates of five-fold serial dilutions of the antiviral compounds were tested in HLT cells using 2-3 different donors. Drug dilutions were prepared in R10 in a 96-well plates. HLT cells were added at a density of 300,000 cells/well and incubated with the compound for at least 1h before infection. Then, MOI 0.1 of VSV*ΔG(Luc)-S virus, generated as previously described with the mutation D614G and a deletion in the last 19 amino acids in the spike (plasmid kindly provided by Dr. Javier García-Pérez, Instituto de Salut Carlos III, Spain) were added to the plates and spinoculated at 1,200g and 37°C for 2h. Cells were then cultured overnight at 37°C in a 5% CO₂ incubator. After, cells were incubated with Britelite plus reagent (Britelite plus kit; PerkinElmer) and then transferred to an opaque black plate. Luminescence was immediately recorded by a luminescence plate reader (LUMIstar Omega). To evaluate cytotoxicity, we used the CellTiter-Glo^{®} Luminescent kit (Promega), following the manufacturer's instructions. Data was normalized to the mock-infected control, after which EC₅₀ and CC₅₀ values were calculated using Graph-Pad Prism 7.

### Results

### 1-Targeting the RBD-ACE2 interface for a SARS-CoV-2 specific antiviral action

### Molecular dynamics simulations of the RBD-ACE2 interface reveal contact hotspots. RBD

is the critical structure of the virus which role is to attach to the host cell via ACE2. To explore which residues of the RBD are most relevant for establishing the ACE2 interactions, we calculated the contact frequencies in the RBD-ACE2 interface exploiting long scale simulations with 10 µs simulation time (Figure 2A and see methods). Most relevant RBD hotspots are residues that can establish contacts > 90% with the ACE2 and are depicted in Figure 2B on the RBD-ACE2 interface (i.e. GLN493, GLY496, GLN498, THR500, GLY502 and Y505).

Virtual screening. At the virtual screening step, we targeted the RBD-ACE2 interface focusing in particular on areas of high contact frequencies determined in the previous step (see target box in Figure 2B). We screened a curated database of 5849 compounds including FDA- and EMA-approved drugs as well as known drug metabolites against the unbound state of the RBD as well as the ACE2 (see methods). Among the top hits of both screenings, 9 compounds were manually selected adhering to characteristics such as size, features and an extensive bibliography search for SARS-CoV-2 related information (Table 1).

**Table 1. Compounds selected for in vitro inhibition activity assay based on docking or on evidence in literature (highlighted by an asterisk)**

| **Compound name** | **Source** | **Hit in** |
|---|---|---|
| **Nilotinib** | Database | RBD docking |
| **Entrectinib** | Database | RBD docking |
| Rutin | Database | ACE2 docking |
| Diosmin | Database | ACE2 docking |
| Naldemedine | Database | ACE2 docking |
| **Apilimod** | **Bibliography** | * |
| Argatroban | Bibliography | * |
| Otamixaban | Bibliography | * |
| Ivermectin | Bibliography | * |

### 2-Targeting the virus membrane towards an unspecific antiviral action

To validate compounds for an unspecific antiviral action, we investigated their chemical properties as well as monitored their ability to intercalate into the viral membrane using molecular dynamics simulation (see methods). In addition to the selected candidate, we included a LJ-001 as positive control into the list of compounds. LJ-001 has been reported to penetrate the viral membranes from where it inhibits viral entry at a step after virus binding but before virus-cell fusion (Proc. Natl. Acad. Sci. U. S. A. 107, 3157-3162 (2010)). One important chemical feature that is critical for membrane intercalation is sufficient hydrophobicity which can be approximated using the octanol/water partition coefficient (logP). We find that nilotinib, entrectinib, apilimod and the positive control LJ-001 (Figure 3A) which could facilitate their membrane intercalation. Molecular dynamics simulations were used to provide further insights into potential intercalation properties at atomistic resolution for compounds with highest logP values (i.e. nilotinib, positive control LJ-001, entrectinib, apilimod). We find that all top hits are able to penetrate the membrane with the following ranking: entrectinib > apilimod > LJ-001 > nilotinib (Figure 3B-C).

### 3-Proof of concept

### Inhibition of the SARS-Cov-2 entry into the cell

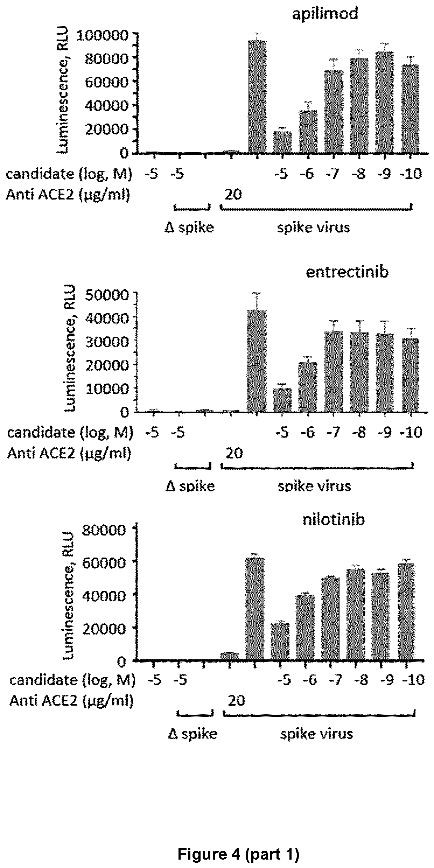

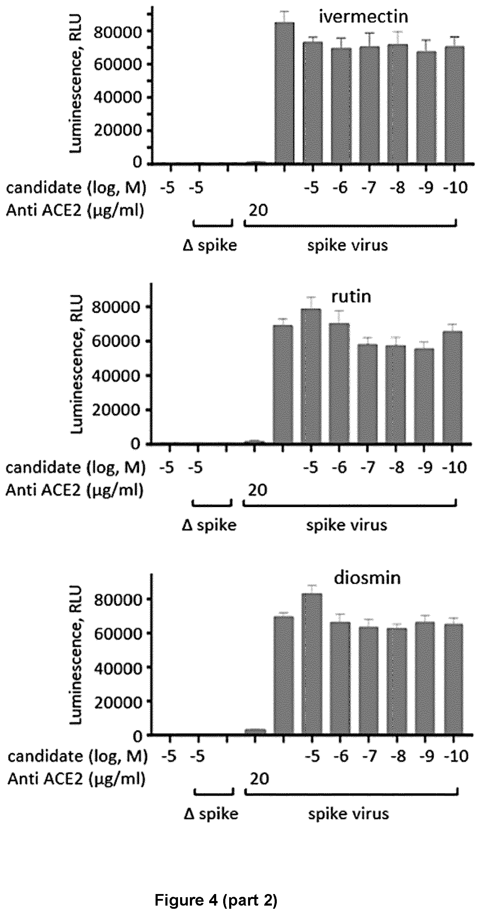

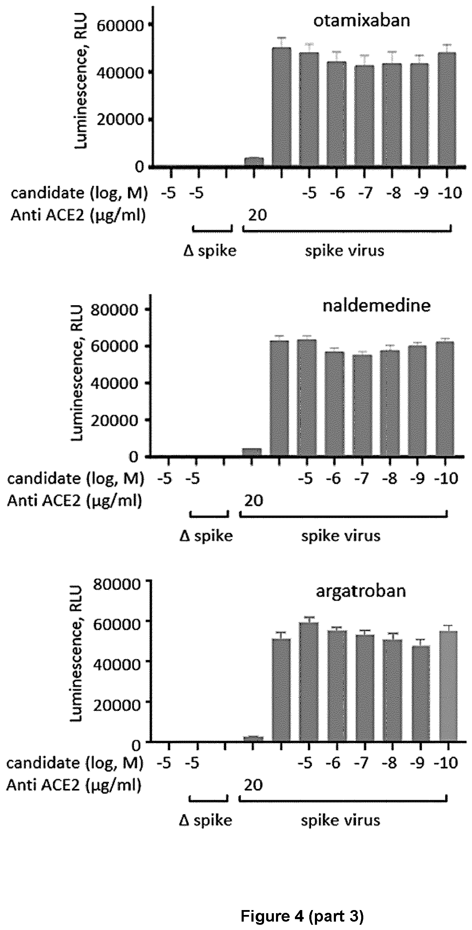

A total of 9 compounds selected from the virtual screening (5) and literature search (4) (see Table 1) were tested in a SARS-CoV-2 pseudovirus assay. Among them, apilimod was previously described by Laura Rivas et al. (Riva, L. et al. Discovery of SARS-CoV-2 antiviral drugs through large-scale compound repurposing. Nature 586, 113-119 (2020)) as a SARS-CoV-2 in vitro infection inhibitor and has been used as a positive control in our experiment. Remarkably, in addition to apilimod, we found two more candidates that show a dose-dependent inhibition of virus infection: entrectinib and nilotinib (Figure 4).

### Specific action on the RBD-ACE2 interface

To further support that the observed *in vitro* effect is specifically mediated via the RBD-ACE2 interface at atomistic resolution, we carried out molecular dynamics simulation for the most promising candidates (i.e. entrectinib, nilotinib as well as apilimod as positive control). For this, we placed three drug molecules randomly distributed in the aqueous phase and free to move around the RBD or ACE2. Preferred binding sites were detected by computing the occupancy map for every compound over the simulation trajectory (see method). The simulation data reveal that all three compounds are able to explore the protein surface and sample promising binding modes at the RBD-ACE2 interface. Interesting is the fact that promising binding modes for all three candidates are found in the similar regions at the RBD and ACE2.

### Antiviral assay in Human Lung Tissue

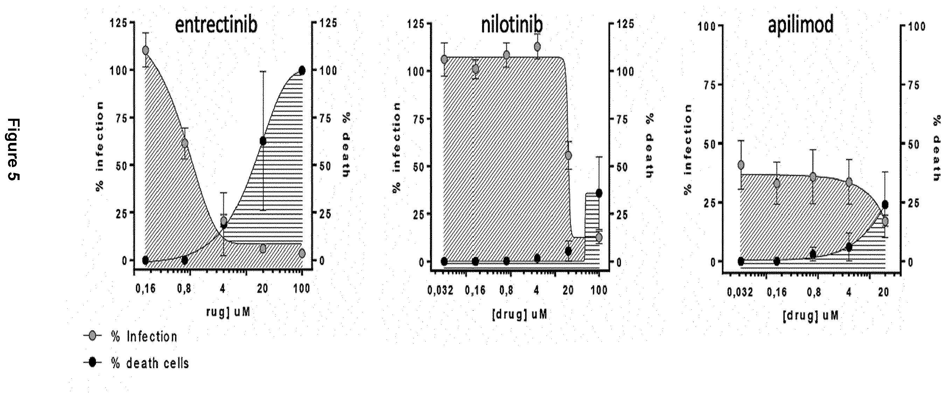

To validate if the observed inhibitory effect of detected candidates (i.e. apilimod, entrectinib and nilotinib) translates into more native conditions, we carried out an antiviral assay in Human Lung Tissue (HTL) cells. Remarkably, entrectinib reveals a potent decrease of cell infection without inducing significant cell death (EC50 < 1 µM) (Figure 5). Nilotinib is also found active but only at high concentrations (EC50 > 15 µM). Interestingly, the positive control apilimod showed 50% infection rate from the beginning which points to a cytostatic effect of apilimod in our HTL setup which was reproduced in several repetitive experiments.

### Non-specific antiviral action

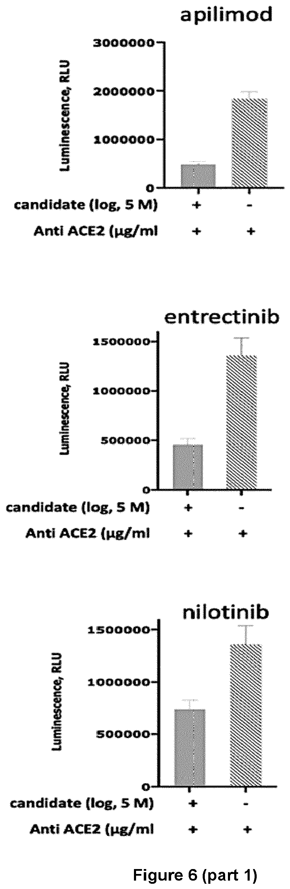

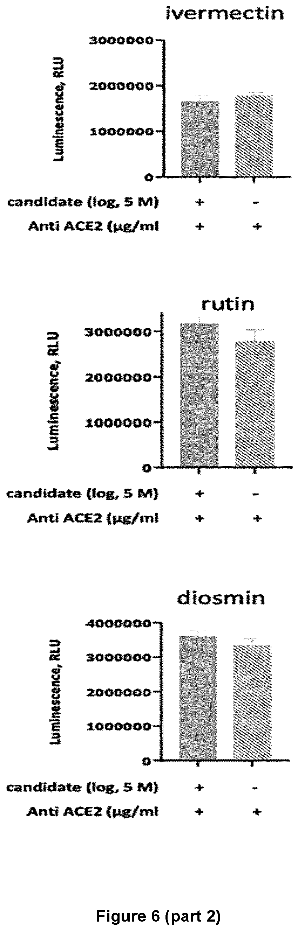

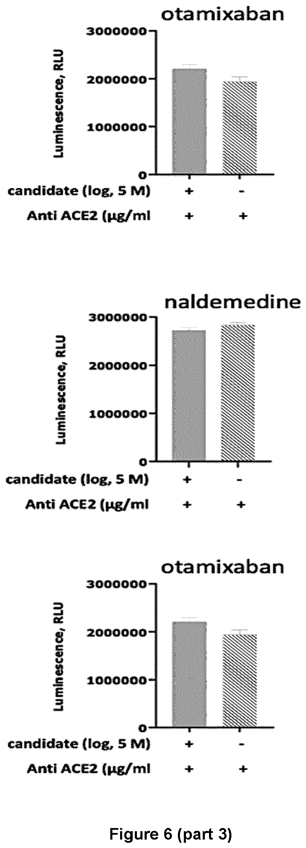

As outlined above, compounds that combine specific with unspecific antiviral effects can be superior in facing virus evolution and the emergence of new variants. The in silico characterization of selected hits in this study suggest that apilimod, entrectinib and nilotinib are able to interfere with the viral membrane (Figure 3B-C). Given this potentially additional mechanism, these compounds should be able to inhibit to some extent other envelope viruses. To interrogate this possibility, we carried out a cell entry assay for the VSV.G virus - an envelope virus lacking the SARS-CoV spike protein (Figure 6). Interestingly, we find that apilimod, entrectinib and nilotinib are able to partially inhibit the VSV.G virus. This finding suggests that in addition to a SARS-CoV-2 specific effect, these compounds elicit also unspecified antiviral action.

The above results demonstrate that entrectinib is a potent inhibitor for SARS-CoV-2 in vitro and in human lung tissue. Interestingly, the results indicate that this antiviral effect is mediated by specific and non-specific effects.

### Citation List

Cagno, V., Magliocco, G., Tapparel, C. & Daali, Y. The tyrosine kinase inhibitor nilotinib inhibits SARS-CoV-2 in vitro. Basic Clin. Pharmacol. Toxicol. 128, 621-624 (2021).

Sterling, T. & Irwin, J. J. ZINC 15 - Ligand Discovery for Everyone. J. Chem. Inf. Model. 55, 2324-2337 (2015).

Schrödinger Release 2021-1: LigPrep, Schrödinger, LLC, New York, NY, 2021.

O'Boyle, N. M. et al. Open Babel: An open chemical toolbox. J. Cheminformatics 3, 33 (2011).

Trott, O. & Olson, A. J. AutoDock Vina: improving the speed and accuracy of docking with a new scoring function, efficient optimization and multithreading. J. Comput. Chem. 31, 455-461 (2010).

Forli, S. et al. Computational protein-ligand docking and virtual drug screening with the AutoDock suite. Nat. Protoc. 11, 905-919 (2016).

D. E. Shaw Research. Molecular Dynamics Simulations Related to SARS-CoV-2. D. E. Shaw Research Technical Data. http://www.deshawresearch.com/resources_sarscov2.html (2020).

Humphrey, W., Dalke, A. & Schulten, K. VMD: Visual molecular dynamics. J. Mol. Graph. 14, 33-38 (1996).

Doerr S, Harvey MJ, Noé F, De Fabritiis G. HTMD: High-Throughput Molecular Dynamics for Molecular Discovery. J Chem Theory Comput. 2016;12(4):1845-1852

Klauda JB, Venable RM, Freites JA, et al. Update of the CHARMM all-atom additive force field for lipids: validation on six lipid types. J Phys Chem B. 2010;114(23):7830-7843

Huang J, Rauscher S, Nawrocki G, et al. CHARMM36m: an improved force field for folded and intrinsically disordered proteins. Nat Methods. 2017;14(1):71-73

Harvey MJ, Giupponi G, Fabritiis GD. ACEMD: Accelerating Biomolecular Dynamics in the Microsecond Time Scale. J Chem Theory Comput. 2009;5(6):1632-1639

Casalino L, Gaieb Z, Goldsmith JA, et al. Beyond Shielding: The Roles of Glycans in the SARS-CoV-2 Spike Protein. ACS Cent Sci. 2020;6(10):1722-1734

Grau-Expósito, J. et al. Novel Human Lung Tissue Model for the Study of SARS-CoV-2 Entry, Inflammation and New Therapeutics. bioRxiv, 2020 https://doi.org/10.1101/2021.04.21.440731

Whitt, M.A., Generation of VSV pseudotypes using recombinant DeltaG-VSV for studies on virus entry, identification of entry inhibitors, and immune responses to vaccines. JVirol Methods, 2010. 169(2): p. 365-74.

Wolf, M. C. et al. A broad-spectrum antiviral targeting entry of enveloped viruses. Proc. Natl. Acad. Sci. U. S. A. 107, 3157-3162 (2010).

Riva, L. et al. Discovery of SARS-CoV-2 antiviral drugs through large-scale compound repurposing. Nature 586, 113-119 (2020).

DESRES-ANTON-10905033, http://www.deshawresearch.com/resources sarscov2.html

## Claims

1. A compound of formula (I), or a pharmaceutically or veterinary acceptable salt thereof, for use in treating infections caused by enveloped viruses.

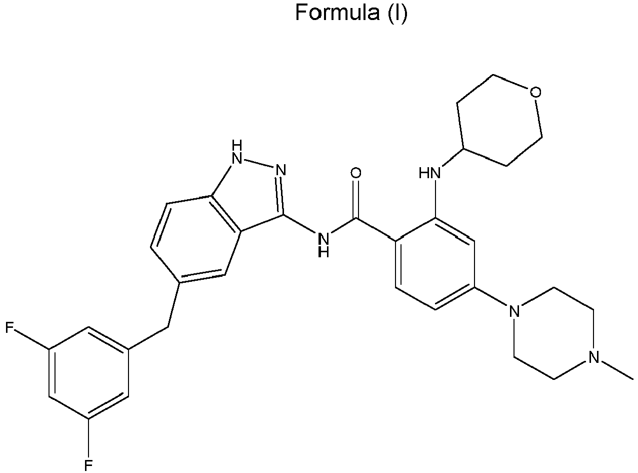

2. A pharmaceutical or veterinary composition comprising a therapeutically effective amount of a compound of formula (I), or a pharmaceutically or veterinary acceptable salt thereof, together with one or more pharmaceutically or veterinary acceptable excipients or carriers, for use in treating infections caused by enveloped viruses.

3. The compound for use according to claim 1 or the pharmaceutical or veterinary composition for use according to claim 2, wherein the enveloped virus is a coronavirus.

4. The compound or the pharmaceutical composition for use according to claim 3, wherein the coronavirus is SARS-CoV-2.

5. The compound for use according to any one of claims 1, 3-4 or the pharmaceutical or veterinary composition for use according to any one of claims 2-4, wherein the treatment is, at least in part, mediated by disruption of the virus membrane.

6. The compound for use according to any one of claims 1, 3-5 or the pharmaceutical or veterinary composition for use according to any one of claims 2-5, wherein the treatment comprises administering the compound to a patient that is refractory to treatment with a previous antiviral drug.

7. The compound for use according to any one of claims 1, 3-6 or the pharmaceutical composition for use according to any one of claims 2-6, wherein the previous antiviral drug is selected from the group consisting of remdesivir, ivermectin, plitidepsin, lopinavir, ritonavir, nitazoxanide, chloroquine, hydroxychloroquine and PF-07321332.

8. The compound for use according to any one of claims 1, 3-7 or the pharmaceutical composition for use according to any one of claims 2-7, wherein the treatment comprises administering a dose of the compound which ranges from 100 to 1000 mg daily.

9. A combination comprising:
a) compound of formula (I) or pharmaceutically or veterinary acceptable salt thereof, and
b) a second antiviral pharmaceutical or veterinary active ingredient that is different from the compound of formula (I) or pharmaceutically or veterinary acceptable salt thereof.

10. A single pharmaceutical or veterinary composition which comprises a therapeutically effective amount of:
a) a compound of formula (I), or a pharmaceutically or veterinary acceptable salt thereof; and
b) a second antiviral pharmaceutical or veterinary active ingredient that is different from the compound of formula (I) or pharmaceutically or veterinary acceptable salt thereof, together with one or more pharmaceutically or veterinary acceptable excipients or carriers.

11. A package or kit of parts comprising:
i) a first pharmaceutical or veterinary composition which comprises a therapeutically effective amount
of a compound of formula (I), or a pharmaceutically or veterinary acceptable salt thereof, together with one or more pharmaceutically or veterinary acceptable excipients or carriers; and
ii) a second pharmaceutical or veterinary composition which comprises a therapeutically effective amount of a second antiviral pharmaceutical or veterinary active ingredient or a pharmaceutically or veterinary acceptable salt thereof, together with one or more pharmaceutically or veterinary acceptable excipients or carriers;
wherein compositions i) and ii) are separate compositions.

12. The combination according to claim 9 or the pharmaceutical or veterinary composition according to claim 10 or the package or kit of parts according to claim 11, wherein the second antiviral pharmaceutical or veterinary active ingredient is selected from viral RNA polymerase inhibitors, viral protein synthesis inhibitors, inhibitors of viral entry, immunomodulators, and inhibitors of the cytoplasmic replication.

13. A combination as defined in any one of claims 9 or 12, a single pharmaceutical or veterinary composition as defined in any one of claims 10 or 12, or a package or kit of parts as defined in any one of claims 11 or 12, for use in treating infections caused by enveloped viruses, in particular, SARS-CoV-2.

14. A compound of formula (I), or a pharmaceutically or veterinary acceptable salt thereof, for use in combination therapy in the treatment of infections caused by an enveloped virus, in particular, SARS-CoV-2, wherein the compound of formula (I), or a pharmaceutically or veterinary acceptable salt thereof, is to be administered simultaneously, concurrently, separately or sequentially within a therapeutic interval with a second antiviral pharmaceutical or veterinary active ingredient or a pharmaceutically or veterinary acceptable salt thereof that is different from the compound of formula (I).
